# EUROPEAN PATENT APPLICATION

(11) **EP 4 736 670 A1**
(43) Date of publication of application: **06.05.2026**
(21) Application number: 26152337.7
(22) Date of filing: 05.11.2020
(51) Int. Cl.: A23L 33/13, A61K 9/1276, A61K 31/7105, A61K 35/20, A61P 3/10

(54) **BOVINE MILK-ISOLATED POWDERED EXOSOMES, NUTRITIONAL COMPOSITIONS AND METHODS**

(30) Priority: 08.11.2019 EP 19382978
(62) Divisional of application: 20812204.4
(71) Applicant: ABBOTT LABORATORIES, Abbott Park, IL 60064-3500 (US)
(72) Inventor: LÓPEZ PEDROSA, José María, 18190 Cenes de la Vega Granada (ES); GARCÍA MARTÍNEZ, Jorge, 18100 Armilla Granada (ES)
(74) Representative: Boult Wade Tennant LLP

(57) **Abstract**

Bovine milk-isolated powdered exosomes comprise intact exosomes. Nutritional compositions comprise protein, carbohydrate, and/or fat, and exosomes isolated from bovine milk. A method of preparing powdered exosomes comprises centrifuging bovine milk to form a lipid fraction top layer, a whey fraction middle layer, and a first pellet of cells and debris, separating the whey fraction and centrifuging the separated whey fraction to remove additional fat, casein aggregates and debris and form a substantially clear whey fraction, microfiltering the substantially clear whey fraction to remove residual debris, centrifuging the microfiltered whey fraction to obtain a pellet containing exosomes, incubating the exosomecontaining pellet in aqueous medium to dissolve the pellet without disrupting exosome membranes to provide an exosome suspension, and drying the suspension to obtain the powdered exosomes. Methods of lowering a risk of developing, or treating, insulin resistance, prediabetes, or diabetes in a subject employ exosomes isolated from bovine milk.

## Description

### FIELD OF THE INVENTION

The present invention relates to bovine milk-isolated powdered exosomes and to nutritional compositions containing protein, carbohydrate, and/or fat, and exosomes isolated from bovine milk. The present invention also relates to methods of preparing the bovine milk-isolated powdered exosomes, methods of preparing the nutritional compositions, and methods of lowering a risk of developing, or treating, insulin resistance, prediabetes, or diabetes in a subject by administering powdered exosomes or powdered nutritional compositions.

### BACKGROUND OF THE INVENTION

Bovine milk contains exosomes which are extracellular bilayer vesicles. The bovine milk exosomes contain bioactive agents such as enzymatic and non-enzymatic proteins (CD9, CD63, MHC-class II, lactadherin, TSG101 and Hsc70, among others.), nucleic acids (including high amounts of microRNA (miRNA) and messenger RNA (mRNA)) and lipids (phosphatidylethanolamine, phosphatidylserine, phosphatidylcholine and sphingomyelin, among others), that are known to have a role in regulating health and disease. Milk exosomes can be actively taken up by cells to deliver their content, but uptake requires the milk exosome bilayer membrane to be preserved. While the exosome structure protects bioactive agents, known methods of isolation of exosomes from milk can damage the exosome membrane, preventing uptake of the exosomes and resulting in release of bioactive agents and degradation of their bioactivity. For example, agents such as miRNA and other bioactive agents contained within the exosomes are extremely unstable and degrade within seconds of exposure when the bilayer membrane is damaged. Similarly, processing of milk and/or isolated exosomes, and/or extended storage of isolated exosomes, can damage the exosome membrane or otherwise degrade bioactivity. Accordingly, improved methods of isolating bovine milk exosomes and exosomes of improved stability are desirable.

Insulin resistance, prediabetes, and diabetes are conditions of glucose dysregulation. Lifestyle modifications and insulin sensitizers are among the treatment strategies available, however these often fail and insulin therapy can become necessary. Dietary management can improve metabolic health and lead to weight loss. Planning for weight maintenance over time, sustaining and improving muscle mass, and taking vitamins and minerals can improve quality of life. Unfortunately, in many cases, lifestyle modifications are inadequate to fully treat such conditions. New compositions and methods for improving insulin sensitivity are therefore needed.

### SUMMARY OF THE INVENTION

In one embodiment, the invention is directed to bovine milk-isolated powdered exosomes comprising intact exosomes. An intact exosome is one in which the bilayer membrane is undamaged and the contents of the exosome are retained within the exosome.

In another embodiment, the invention is directed to a nutritional composition which comprises protein, carbohydrate, and/or fat, and exosomes isolated from bovine milk, wherein the exosomes are provided by addition of the powdered exosomes of the invention. In specific embodiments, the nutritional composition is in the form of a powder, and in additional specific embodiments, the nutritional composition is in the form of a liquid. In further embodiments, the nutritional composition comprises protein, carbohydrate, fat, and exosomes isolated from bovine milk, wherein the exosomes are provided by addition of the powdered exosomes of the invention.

In an additional embodiment, the invention is directed to a method of preparing powdered exosomes. The method comprises centrifuging bovine milk to form a lipid fraction top layer, a whey fraction middle layer, and a first pellet of cells and debris, separating the whey fraction and centrifuging the separated whey fraction to remove additional fat, casein aggregates and debris and form a substantially clear whey fraction, microfiltering the substantially clear whey fraction to remove residual debris, centrifuging the microfiltered whey fraction to obtain a pellet containing exosomes, incubating the exosome-containing pellet in aqueous medium to dissolve the pellet without disrupting exosome bilayer membranes to provide an exosome suspension, and drying the suspension to obtain the powdered exosomes.

In another embodiment, the invention is directed to a method of preparing a powdered nutritional composition. The method comprises dry blending a powder composition comprising protein, fat, and carbohydrate with powdered exosomes isolated from bovine milk.

In further embodiments, the invention is directed to a method of lowering a risk of developing insulin resistance, prediabetes, or diabetes in a subject, or treating insulin resistance, prediabetes, or diabetes in a subject. The method comprises administering to a subject at risk of developing, or having, insulin resistance, prediabetes, or diabetes the exosomes according to the invention or the nutritional composition of the invention, wherein the administered amount of exosomes is effective to lower the risk of development of, or treat, respectively, insulin resistance, prediabetes, or diabetes.

The bovine milk-isolated exosomes of the invention are advantageous in providing bioactive agents in a convenient and stable form, and, in certain embodiments, in a nutritional composition. The use of the bioactive agents for therapeutic benefits is therefore facilitated. These and additional objects and advantages of the invention will be more fully apparent in view of the following detailed description.

### BRIEF DESCRIPTION OF THE DRAWINGS

The embodiments set forth in the drawings are illustrative of certain aspects of the invention and exemplary in nature and are not intended to limit the invention defined by the claims, wherein;
**FIGS. 1A-1I** illustrate images from a transmission electron microscope (TEM) at a magnification of 880x (**FIGS. 1A-1C**), at a magnification of 10,000x (**FIGS. 1D-1F**), and at a magnification of 25,000x (**FIGS. 1G-1I**). **FIGS. 1A****,** **1D** and **1G** show fresh exosomes, **FIGS. 1B****,** **1E** and **1H** show frozen-thawed exosomes, and **FIGS. 1C****,** **1F** and **1I** show powdered exosomes according to an embodiment of the invention, all with uranyl acetate staining, as described in Example 1.
**FIG. 2A** illustrates relative expression of micro RNA (miRNA) in fresh exosomes and powdered exosomes according to an embodiment of the invention, respectively, as described in Example 1. **FIG. 2B** illustrates the content of several microRNA (miRNA) associated with insulin regulation in nutritional compositions supplemented with freeze dried raw milk and freeze dried powdered exosomes according to an embodiment of the invention, respectively, as described in Example 1.
**FIG. 3** illustrates glucose uptake in muscle cells incubated with increasing concentrations of freeze-dried exosomes or sonicated exosomes, respectively, for 24 hours, as described in Example 2.
**FIG. 4** illustrates GLUT-4 transporter protein levels in muscle cells incubated with freeze-dried exosomes or sonicated exosomes, respectively, for 24 hours, as described in Example 2.
**FIG. 5** illustrates GLUT-4 mRNA levels in muscle cells incubated with freeze-dried exosomes or sonicated exosomes, respectively, for 24 hours, as described in Example 2.
**FIG. 6** illustrates GLUT-4 gene promoter activity in muscle cells incubated with increasing concentrations of freeze-dried exosomes or sonicated exosomes, respectively, for 24 hours, as described in Example 2.

### DETAILED DESCRIPTION

Specific embodiments of the invention are described herein. The invention can, however, be embodied in different forms and should not be construed as limited to the embodiments set forth herein. Rather, these embodiments are provided to illustrate more specific features of certain aspects of the invention to those skilled in the art.

The terminology as set forth herein is for description of the embodiments only and should not be construed as limiting the disclosure as a whole. All references to singular characteristics or limitations of the present disclosure shall include the corresponding plural characteristic or limitation, and vice versa, unless otherwise specified or clearly implied to the contrary by the context in which the reference is made. Unless otherwise specified, "a," "an," "the," and "at least one" are used interchangeably. Furthermore, as used in the description and the appended claims, the singular forms "a," "an," and "the" are inclusive of their plural forms, unless the context clearly indicates otherwise.

To the extent that the term "includes" or "including" is used in the description or the claims, it is intended to be inclusive of additional elements or steps, in a manner similar to the term "comprising" as that term is interpreted when employed as a transitional word in a claim. Furthermore, to the extent that the term "or" is employed (e.g., A or B), it is intended to mean "A or B or both." When the "only A or B but not both" is intended, then the term "only A or B but not both" will be employed. Thus, use of the term "or" herein is the inclusive, and not the exclusive use. When the term "and" as well as "or" are used together, as in "A and/or B" this indicates A or B as well as A and B.

The powdered exosomes, the nutritional compositions and the methods described in the present disclosure can comprise, consist of, or consist essentially of any of the elements and steps as described herein.

All ranges and parameters, including but not limited to percentages, parts, and ratios disclosed herein are understood to encompass any and all sub-ranges subsumed therein, and every number between the endpoints. For example, a stated range of "1 to 10" should be considered to include any and all sub-ranges beginning with a minimum value of 1 or more and ending with a maximum value of 10 or less (e.g., 1 to 6.1, or 2.3 to 9.4), and to each integer (1, 2, 3, 4, 5, 6, 7, 8, 9, and 10) contained within the range.

Any combination of method or process steps as used herein can be performed in any order, unless otherwise specified or clearly implied to the contrary by the context in which the referenced combination is made.

All percentages are percentages by weight unless otherwise indicated.

The term "bovine milk-isolated exosomes" as used herein, unless otherwise specified, refers to exosomes that have been substantially separated from other bovine milk components such as lipids, cells, and debris, and are concentrated in an amount higher than that found in bovine milk. Milk exosomes are small solid particles that are "dissolved" in bovine milk and account for a minor percentage of milk's total solids. Isolation of the exosomes as described herein produces a fluid in which the exosomes originally present in milk are concentrated. As will be apparent, the powdered exosomes may also contain other milk solids that share a size with the milk exosomes and are co-isolated with the exosomes (i.e., caseins and other whey proteins).

The term "powdered exosomes" as used herein, unless otherwise specified, refers to a dry powder that contains exosomes which have been isolated from bovine milk. The exosomes are dried to form a dry powder. In specific examples, the isolated exosomes have been freeze-dried to form the powdered exosomes. As the isolated fluid containing the exosomes also contains co-isolated milk solids as described above, the powdered exosomes also contain such other milk solids in the resulting powder.

Importantly, the powdered exosomes of the invention comprise intact exosomes. An intact exosome is one in which the bilayer membrane is undamaged and the contents of the exosome are retained within the exosome.

As stated earlier, milk contains exosomes which contain bioactive agents that can improve health. More specifically, bovine milk contains, for example, multiple miRNAs for promoting healthy function of diverse organs, tissues, and systems ranging from muscle to bone and fat to skin, brain, liver, gut, and the vasculature. However, factors such as miRNAs tend to degrade quickly, and such beneficial functions are lost. Milk exosomes provide a protective environment for miRNAs, but current techniques for isolating exosomes often lead to damage to the exosome membrane. The present invention, in contrast, provides intact isolated exosomes in powder form.

Generally, the bovine milk-isolated powdered exosomes are obtained from bovine milk using gentle procedures which do not disrupt the exosome bilayer membrane, thereby leaving the exosome intact and the bioactive agents contained within the exosome structure. In one embodiment, isolated and concentrated exosomes are provided in a suspension and the suspension is dried to provide the powdered exosomes. Various methods may be employed to isolate exosomes with care being exercised to avoid disruption of the bilayer membrane. Fresh bovine milk and/or thawed frozen bovine milk may be employed as a source of exosomes. In specific embodiments, the step of isolating the exosomes comprises performing the isolation immediately upon obtaining milk from a bovine. In additional embodiments, the step of isolating the exosomes comprises performing the isolation within about 1 day, or about 2 days, or about 3 days, or about 4 days, or about 5 days or about 6 days, or about 7 days from the time of obtaining the milk from a bovine. In specific embodiments, the exosomes are isolated within about 10 days, or within about 14 days from the time of obtaining milk from a bovine. In additional embodiments, the bovine milk may be frozen and then thawed for processing as described herein for isolating exosomes, with the bovine milk preferably having been frozen within about 1 day, or about 2 days, or about 3 days, or about 4 days, or about 5 days or about 6 days, or about 7 days from the time of obtaining the milk from a bovine. Thawed milk is preferably processed immediately upon thawing. In a specific embodiment, fresh bovine milk is subjected to the processing as described herein within about 5 days of obtaining the milk from a bovine, or thawed bovine milk which is subjected to the processing as described herein is thawed from bovine milk that was frozen within about 5 days of obtaining the milk from a bovine.

In one embodiment, exosomes are isolated from bovine milk by centrifuging bovine milk to form a lipid fraction top layer, a whey fraction middle layer, and a first pellet of cells and debris. The whey fraction is separated from the lipid fraction and the first pellet and is subjected to further centrifugation to produce a substantially clear whey fraction. Additional fat, casein aggregates, and debris, for example, are removed to produce the substantially clear whey fraction. The substantially clear whey fraction is then microfiltered to remove residual debris. The microfiltered whey fraction is then subjected to additional centrifugation to obtain a second pellet containing exosomes. This second pellet is then suspended in aqueous medium to dissolve the second pellet without disrupting the bilayer membrane of exosomes therein and to provide an exosome suspension. It is important to suspend the second pellet in a gentle manner which does not disrupt the exosome membrane. In one specific embodiment, the second pellet is incubated for an extended period of time, for example at least 6 hours, at least 8 hours, at least 10 hours or at least 12 hours, and up to 18, 24, 30 or 36, or more, hours, in an aqueous medium such as sterile phosphate buffered saline (PBS) or sterile molecular biology water. For example, an orbital shaker at low speed, i.e., not more than than 500 rpm, may be employed. Once the pellet is fully suspended, the suspension is dried to obtain the powdered exosomes. Again, the drying step must be conducted with care to avoid disruption of the bilayer membrane of the exosomes. In one specific embodiment, the drying step comprises freeze drying.

In specific embodiments of the invention, exosome isolation comprises centrifugation at specific speeds, times and/or temperatures. Centrifugation times and speeds as described herein provide for intact exosome isolation, as centrifuging can cause exosome membrane damage if performed too forcefully. In specific embodiments, bovine milk is centrifuged below about 15,000 G, for example, at about 12,000 G, for example, at about 4°C for about 15 minutes, to obtain a whey layer formed between a top layer of fat (lipid) and the cell debris pellet. In specific embodiments, the whey fraction is centrifuged two more times, again under conditions to maintain the exosomes in intact form, for example, at about 12,000 G and about 4°C for about 30 minutes, each time to remove additional fat and/or debris. A substantially clear whey fraction is obtained. The substantially clear whey fraction is microfiltered, for example, using a 0.22 µm filter of hydrophilic material such as polyether sulfone having low protein retention, and the microfiltered whey is then centrifuged at 100,000 G at 4°C for about 60 minutes, to form an exosome-containing pellet. In specific embodiments, the exosome-containing pellet is dissolved in an orbital the shaker by incubation for at least about 12 hours, or from about 12 hours to about 36 hours, or from about 15 hours to about 30 hours, or from about 18 hour to about 24 hours. The dissolution is conducted under conditions which maintain the exosomes in intact form, i.e., the bilayer membrane is not damaged and the contents of the exosomes are maintained therein.

The exosomes are then dried, again, under conditions which maintain the exosomes in intact form. In specific embodiments of the invention, the isolated exosomes are dried by freeze-drying to form powdered exosomes under conditions which maintain the exosomes in intact form. In specific embodiments of the invention, the step of freeze-drying comprises exposing the exosome suspension to a temperature of -80°C and a vacuum of less than 0.3 mbar for a sufficient time period. In specific embodiments, depending on the amount of liquid to be freeze-dried and the features of the freeze-drying equipment, the time may vary from about 5 to about 40 hours, more specifically from about 10 to about 30 hours, or from about 15 to about 25 hours. Importantly, the process should fully dry the exosomes. In a specific embodiment as described in the examples, at a temperature of -80°C and a vacuum of less than 0.3 mbar, the time period for freeze-drying was 24 hours or more.

Additional embodiments of the method comprise variations of time, temperature, and pressure for freeze-drying. In additional embodiments of the method, the milk exosomes are: kept at a temperature of at least about -50°C, or at least about -60°C, or at least about -70°C, or at least about -80°C; subjected to a vacuum of less than about 0.3 mbar, or less than about 0.2 mbar, or less than about 0.1 mbar; and kept under these conditions for at least about 5, 10, 15, 20, 25, 30, 35 or 40 hours.

Powdered exosomes produced according to such methods comprise intact exosomes, i.e., exosomes in which the bilayer membrane is not ruptured and/or otherwise degraded and the contents of the exosomes are retained therein. In specific embodiments, at least about 50 wt % of the exosomes in the powder are intact. In further embodiments, at least about 55, 60, 65, 70, 75, 80, 85, 90, or 95 wt% of the exosomes in the powder are intact. In specific embodiments, at least 50 wt % of the exosomes in the powder remain intact after storage of the composition for at least two months at a temperature of 20°C. In further embodiments, at least about 55, 60, 65, 70, 75, 80, 85, 90, or 95 wt% of the exosomes in the powder remain intact after storage of the composition for at least two months at a temperature of 20°C. Additionally, in certain embodiments, the powdered exosomes exhibit good stability at higher temperature conditions. For example, in a specific embodiment, after storage at 37°C for 3 weeks, the bovine milk-isolated powdered exosomes comprise a greater amount of miRNA than fresh milk exosomes stored at 37°C for 3 weeks, thus evidencing a greater resistance to bilayer membrane rupture and/or other degradation under such storage conditions, as compared with fresh milk exosomes. In another specific embodiment, after storage at 55°C for 1 week, the bovine milk-isolated powdered exosomes comprise a greater amount of miRNA than fresh milk exosomes stored at 55°C for 1 week, thus further evidencing a greater resistance to membrane rupture and/or other degradation under such storage conditions, as compared with fresh milk exosomes. This stability of the powdered exosomes is a significant improvement in providing the benefits of the active agent(s) therein in a product for consumer distribution and consumption.

In specific embodiments, greater than 90% of the isolated exosomes are from about 10 nanometers to about 250 nanometers in diameter, or from about 20 to 200 nm in diameter, or from about 50 to 150 nm in diameter.

The powdered exosomes of the invention are particularly suitable for use as a component of a nutritional composition. The nutritional composition may be in powdered form or in liquid form, and comprise bovine milk-isolated exosomes, wherein the exosomes are provided by addition of the powdered exosomes of the invention as described herein. In one embodiment of a powdered nutritional composition, the powdered exosomes are dry blended with one or more dry components of the nutritional composition. In additional embodiments of nutritional compositions of the invention, a liquid nutritional composition comprises a liquid reconstituted from a powdered nutritional composition as described herein. In additional embodiments, a liquid nutritional composition is manufactured in ready-to-drink form, for example, as an emulsion, with the powdered exosomes combined with one or more additional nutritional composition components during the manufacturing process. Such compositions in powder or liquid form can be easily orally administered to obtain the benefits of the bioactive agent(s) in the exosomes.

Specific embodiments of nutritional compositions described herein include a protein, a carbohydrate, and/or a fat, and bovine milk-isolated exosomes wherein the exosomes are provided by addition of the powdered exosomes of the invention. The bovine milk-isolated exosomes may be included in the nutritional compositions in any desired amount effective to provide nutritional or therapeutic benefit. In a specific embodiment, the nutritional compositions comprise from about 0.001 to about 10 wt % of the bovine milk-isolated exosomes, or, more specifically, from about 0. 1 to about 5 wt %, of the bovine milk-isolated exosomes, based on the weight of the composition.

A wide variety of sources and types of protein, carbohydrate, and fat can be used in embodiments of nutritional compositions described herein.

In specific embodiments of the nutritional composition, protein comprises from about 1 wt% to about 30 wt% of the nutritional composition. In more specific embodiments, the protein comprises from about 1 wt% to about 25 wt% of the nutritional composition, including about 1 wt% to about 20 wt%, about 1 wt% to about 15 wt%, about 1 wt% to about 10 wt%, about 5 wt% to about 10 wt%, or about 10 wt% to about 20 wt% of the nutritional composition. In additional specific embodiments, the protein comprises from about 1 wt% to about 5 wt% of the nutritional composition. In additional, specific embodiments, the protein comprises from about 20 wt% to about 30 wt% of the nutritional composition.

In specific embodiments, one or more sources of protein are used in the nutritional composition. For example, the source of protein can include, but is not limited to, intact, hydrolyzed, and/ or partially hydrolyzed protein, which can be derived from a suitable source such as milk (e.g., casein, whey), animal (e.g., meat, fish), cereal (e.g., rice, corn), vegetable (e.g., soy, pea), and combinations thereof. More particular examples of sources of protein used in specific embodiments of the nutritional composition include, but are not limited to, whey protein concentrate, whey protein isolate, whey protein hydrolysate, acid casein, sodium caseinate, calcium caseinate, potassium caseinate, casein hydrolysate, milk protein concentrate, milk protein isolate, milk protein hydrolysate, nonfat dry milk, condensed skim milk, soy protein concentrate, soy protein isolate, soy protein hydrolysate, pea protein concentrate, pea protein isolate, pea protein hydrolysate, collagen protein, collagen protein isolate, rice protein, potato protein, earthworm protein, and/or insect protein, and combinations of two or more thereof. More specific embodiments comprise milk protein concentrate and/or soy protein isolate.

The source of protein can also include a mixture of amino acids (often described as free amino acids) known for use in nutritional products or a combination of such amino acids with the intact, hydrolyzed, and/or partially hydrolyzed proteins described herein. The amino acids can be naturally occurring or synthetic amino acids.

In specific embodiments, the nutritional compositions contain β-hydroxy-β-methylbutyrate (HMB). HMB is a naturally occurring short chain fatty acid metabolite of leucine that is known for use in a variety of nutritional products and supplements. Any source of HMB is suitable for use herein, including, but not limited to, the free acid, a salt, including an anhydrous salt, an ester, a lactone, or other product forms that otherwise provide a bioavailable form of HMB in the nutritional composition. Non-limiting examples of suitable salts of HMB for use herein include HMB salts, hydrated or anhydrous, of sodium, potassium, magnesium, chromium, calcium, or other non-toxic salt form. In a specific embodiment, the HMB is provided by calcium HMB monohydrate. In specific embodiments, the nutritional compositions may comprise from about 0.01 to about 10 wt % HMB. In a more specific embodiment, the nutritional compositions comprise from about from about 0.1% to about 7.0%, or more specifically, from about 0.1% to about 5.0%, HMB. In further embodiments, the nutritional compositions provide from about 1 to 3 grams, or more specifically, from about 1.5 to 3 grams, HMB per 237 ml serving.

In specific embodiments of the nutritional composition, carbohydrate is present in an amount from about 5 wt% to about 75 wt% of the nutritional composition. In more specific embodiments, the carbohydrate is present in an amount from about 5 wt% to about 70 wt% of the nutritional composition, including about 5 wt% to about 65 wt%, about 5 wt% to about 50 wt%, about 5 wt% to about 40 wt%, about 5 wt% to about 30 wt%, about 5 wt% to about 25 wt%, about 10 wt% to about 65 wt%, about 20 wt% to about 65 wt%, about 30 wt% to about 65 wt%, about 40 wt% to about 65 wt%, or about 15 wt% to about 25 wt%, of the nutritional composition.

Carbohydrates used in specific embodiments of a nutritional composition are simple, complex, or a combination thereof. Non-limiting examples of a source of carbohydrate suitable for use in specific embodiments of a nutritional composition described herein include human milk oligosaccharides (HMOs), maltodextrin, hydrolyzed starch, glucose polymers, corn syrup, corn syrup solids, rice-derived carbohydrates, sucrose, glucose, lactose, honey, sugar alcohols, isomaltulose, sucromalt, pullulan, potato starch, galactooligosaccharides, oat fiber, soy fiber, corn fiber, gum arabic, sodium carboxymethylcellulose, methylcellulose, guar gum, gellan gum, locust bean gum, konjac flour, hydroxypropyl methylcellulose, tragacanth gum, karaya gum, gum acacia, chitosan, arabinoglactins, glucomannan, xanthan gum, alginate, pectin, low methoxy pectin, high methoxy pectin, cereal beta-glucans, carrageenan, psyllium, digestion-resistant carbohydrates such as digestion-resistant maltodextrins, digestion-resistant starch, slowly digestible carbohydrates, inulin, fructooligosaccharides, and combinations of two or more thereof.

In specific embodiments, the nutritional composition comprises fat in an amount of from about 0.5 wt% to about 30 wt% of the nutritional composition. In certain specific embodiments, the fat comprises from about 1 wt% to about 30 wt% of the nutritional composition, including about 1 wt% to about 20 wt%, about 1 wt% to about 15 wt%, about 1 wt% to about 10 wt%, about 1 wt% to about 5 wt%, about 3 wt% to about 30 wt%, about 5 wt% to about 30 wt%, about 5 wt% to about 25 wt%, about 5 wt% to about 20 wt%, about 5 wt% to about 10 wt%, or about 10 wt% to about 20 wt% of the nutritional composition.

In specific embodiments, the fat of the nutritional composition is one or more of coconut oil, fractionated coconut oil, soy oil (e.g., high oleic soy oil), corn oil, olive oil, safflower oil (e.g., high oleic safflower oil), medium chain triglyceride oil (MCT oil), high gamma linolenic (GLA) safflower oil, sunflower oil (e.g., high oleic sunflower oil), palm oil, palm kernel oil, palm olein, canola oil (e.g., high oleic canola oil), marine oils, fish oils (e.g., tuna oil), algal oils, borage oil, cottonseed oil, fungal oils, eicosapentaenoic acid (EPA), docosahexaenoic acid (DHA), arachidonic acid (ARA), conjugated linoleic acid (CLA), alpha-linolenic acid, interesterified oils, transesterified oils, structured lipids, and combinations thereof. The source of fat can comprise triglycerides, diglycerides, monoglycerides, phospholipids (e.g., lecithin) and/or free fatty acids. Fatty acids can include, but are not limited to, capric acid, lauric acid, myristic acid, palmitic acid, palmitoleic acid, stearic acid, oleic acid, linoleic acid, alpha-linolenic acid, arachidonic acid (ARA), eicopanteanoic acid (EPA), and/or docosahexanoic acid (DHA). Specific embodiments of the composition of the present disclosure include any individual source of fat or a combination of two or more the various sources of fat listed above. More specific embodiments comprise canola oil, high oleic sunflower oil, medium chain triglycerides and/or one or more fatty acids such as linoleic acid, alpha-linolenic acid, ARA, EPA, and/or DHA.

Additional specific embodiments of the nutritional composition comprise one or more components to modify the physical, chemical, aesthetic, or processing characteristics of the nutritional composition or serve as additional nutritional components. Non-limiting examples of additional components include preservatives, emulsifying agents (e.g., lecithin), buffers, sweeteners including artificial sweeteners (e.g., saccharine, aspartame, acesulfame K, sucralose), colorants, flavorants, thickening agents, stabilizers, and so forth.

Specific embodiments of the nutritional composition optionally comprise one or more prebiotic and/or probiotic. The term "prebiotic" as used herein, unless otherwise specified, refers to a non-digestible food ingredient that beneficially affects a subject by selectively stimulating the growth and/or activity of bacteria in a subject's gastrointestinal (GI) tract. Non-digestible, fermentable polysaccharides are examples of prebiotics that can be included in nutritional compositions described herein. The term "probiotic" as used herein, unless otherwise specified, refers to a microorganism such as a bacteria or yeast that survives the digestive process to confer a health benefit on the host. Examples of probiotics that can be included in nutritional compositions described herein, either alone or in combination, include *Bifidobacterium* (*B*.), such as *B*. *breve* M-16V, *B. infantis* Bb02, *B. infantis* M-63, *B. infantis* 35624, *B. lactis* HN019, *B. lactis* Bi07, *B. bifidum, B. longum* BB536, *B. longum* AH1205, *B. longum AH1206,* and B. *animalis,* and *Lactobacillus* (*L*.), such as *L. rhamnosus* GG, *L. rhamnosus* HN001, *L. acidophilus* (LA-5), *L. acidophilus* NCFM, *L. fermentum* CECT5716, *L. reuteri* ATCC55730, *L. reuteri* ATCC PTA-6475, and *L. reuteri* DSM 17938, *Streptococcus thermophilus* (Th4), *Akkermansia, Bacteroides, Enterococcus, Eubacterium, Fecalibacterium, Roseburia,* and/or *Saccharomyces.*

Specific embodiments of the nutritional composition may comprise vitamins and/or related nutrients, non-limiting examples of which include vitamin A, vitamin B12, vitamin C, vitamin D, vitamin E, vitamin K, thiamine, riboflavin, pyridoxine, niacin, folic acid, pantothenic acid, biotin, choline, inositol, and/or salts and derivatives thereof, and combinations thereof.

Specific embodiments of the nutritional composition comprise minerals, non-limiting examples of which include calcium, phosphorus, magnesium, zinc, manganese, sodium, potassium, molybdenum, chromium, iron, copper, and/or chloride, and combinations thereof.

Additional specific embodiments of the nutritional composition of the invention comprise milk protein concentrate and/or soy protein isolate as proteins, canola oil, sunflower oil, medium chain triglycerides and/or DHA as fats, and hydrolyzed corn starch, sucrose and/or maltodextrin as carbohydrates. In further specific embodiments, these nutritional compositions also include oligofructose, mixed tocopherols, and/or *Lactobacillus acidophilus.*

In another specific embodiment, the nutritional composition comprises milk protein concentrate, sodium caseinate, calcium caseinate, and/or soy protein isolate as proteins, corn syrup, corn maltodextrin and/or sugar as carbohydrates, and corn oil and/or canola oil as fats. In another specific embodiment, the nutritional composition comprises milk protein concentrate as protein, corn maltodextrin, fructose, and/or glycerine as carbohydrates, and high oleic safflower oil as fat.

In specific embodiments, when the nutritional composition is a liquid, for example, reconstituted from a powder as described herein or manufactured as a ready-to-drink product, a serving ranges from about 1 ml to about 500 ml, including from about 110 ml to about 500 ml, from about 110 ml to about 417 ml, from about 120 ml to about 500 ml, from about 120 ml to about 417 ml, from about 177 ml to about 417 ml, from about 207 ml to about 296 ml, from about 230 m to about 245 ml, from about 110 ml to about 237 ml, from about 120 ml to about 245 ml, from about 110 ml to about 150 ml, and from about 120 ml to about 150 ml. In specific embodiments, the serving is about 1 ml, or about 100 ml, or about 225 ml, or about 237 ml, or about 500 ml.

In specific embodiments, when the nutritional composition is a powder, for example, a serving size is from about 40 g to about 60 g, such as 45 g, or 48.6 g, or 50 g, to be administered as a powder or to be reconstituted in from about 1 ml to about 500 ml of liquid, such as about 225 ml, or from about 230 ml to about 245 ml.

In specific embodiments, the nutritional composition provides up to about 500 kcal of energy per serving of the nutritional composition, including from about 20 kcal to about 500 kcal, from about 75 kcal to about 500 kcal, from about 150 kcal to about 500 kcal, from about 250 kcal to about 500 kcal, from about 300 kcal to about 500 kcal, or from about 400 kcal to about 500 kcal per serving of the nutritional composition.

In specific embodiments, the nutritional composition has a caloric density of about 0.5 kcal/ml to about 3 kcal/ml. In specific embodiments, the nutritional composition has a caloric density from about 0.5 kcal/ml to about 2.5 kcal/ml, including about 0.5 kcal/ml to about 2 kcal/ml, about 0.5 kcal/ml to about 1.5 kcal/ml, about 0.5 kcal/ml to about 1 kcal/ml, or about 0.5 kcal/ml to about 0.8 kcal/ml. In specific embodiments, the nutritional composition has a caloric density of about 1 kcal/ml to about 3 kcal/ml, including about 1.5 kcal/ml to about 3 kcal/ml, about 2 kcal/ml to about 3 kcal/ml, or about 2.5 kcal/ml to about 3 kcal/ml.

In specific embodiments, the nutritional composition has a caloric density of from about 1 kcal per gram to about 10 kcal per gram, such as about 4.6 kcal per gram. In specific embodiments, the nutritional composition has a caloric density of from about 2 kcal per gram to about 8 kcal per gram, or from about 3 kcal per gram to about 6 kcal per gram.

In specific embodiments, the nutritional composition is in liquid form, reconstituted from a powdered nutritional composition, and has a pH of about 3 to about 9, or from about 6 to about 8.

In specific embodiments of the methods described herein, the powdered nutritional compositions are formed by dry blending the powdered exosomes with powder comprising one or more of protein, fat, and carbohydrate to produce a shelf-stable powdered nutritional composition. The powdered nutritional compositions may be administered in powder form or may be reconstituted with aqueous liquid to form a liquid nutritional composition.

Owing to the intact nature of the exosomes in the powdered form, the exosomes, and compositions to which the powdered exosomes are added, are suitable for use in providing a nutritional or therapeutic benefit based on the bioactive agents in the exosomes, including various miRNA contained in intact exosomes. In specific embodiments, the powdered exosomes, and compositions to which the powdered exosomes are added, are suitable for use in lowering a risk of insulin resistance, prediabetes, or diabetes, and/or for treating insulin resistance, prediabetes, or diabetes. Risk factors for developing insulin resistance, prediabetes, or diabetes can include being overweight, being sedentary or physically active less than 3 times per week, having had gestational diabetes previously, or having an immune disorder. Subjects with insulin resistance, prediabetes, or diabetes are also often obese, hypertensive, have dyslipidemia, and/or have sarcopenia.

In specific embodiments of the methods described herein, the nutritional composition is administered orally or via tube-feeding. In specific embodiments, when the administration is via tube feeding, the tube-feeding is performed through the nose of the subject or directly into the stomach or small intestine of the subject through an incision in the abdomen of the subject.

In specific embodiments, the methods for lowering a risk of insulin resistance, prediabetes, or diabetes, or treating these conditions, comprise administering to a subject the bovine milk-isolated powdered exosomes as described herein or a nutritional composition to which the bovine milk-isolated powdered exosomes as described herein have been added. The nutritional composition may be administered in powder or liquid form, as desired. In specific embodiments, the bovine milk-isolated powdered exosomes or nutritional compositions to which the bovine milk-isolated powdered exosomes as described herein have been added are administered to a subject once or multiple times daily or weekly. In specific embodiments, the nutritional composition is administered to the subject from about 1 to about 6 times per day or per week, or from about 1 to about 5 times per day or per week, or from about 1 to about 4 times per day or per week, or from about 1 to about 3 times per day or per week.

In specific embodiments, the powdered exosomes of the invention are administered, directly or via addition to a nutritional composition, in an effective amount to lower the risk of insulin resistance, prediabetes and/or diabetes in a subject who is at risk of developing one or more of such conditions. In additional specific embodiments, the powdered exosomes of the invention are administered, directly or via a nutritional composition, in an effective amount to treat insulin resistance, prediabetes and/or diabetes in a subject who is diagnosed with one or more of such conditions. In specific embodiments of the methods described herein, a dosage of from about 0.01 to about 10 g of powdered exosomes is administered to a subject, directly or via addition to a nutritional composition. In additional embodiments, a dosage of from about 0.1 to about 10 g, or from about 1 to about 5 g, of powdered exosomes are administered to a subject, directly or via addition to a nutritional composition.

The following examples demonstrate various aspects of the invention.

### EXAMPLES

### Example 1: Production of Powdered Exosomes

This examples describes preparation of powdered exosomes and powdered nutritional compositions comprising exosomes. Exosomes first were isolated from bovine milk and then dried. Exosomes were isolated from bovine milk using the following procedure. Raw unprocessed bovine milk was aliquoted and immediately frozen at -80°C. Aliquots were thawed on ice and subjected to a first centrifugation at about 12,000 G at about 4°C for about 15 minutes to obtain a whey layer formed between a top layer of fat (lipid) and the cell debris pellet. The whey fraction was transferred to a clean tube and centrifuged two more times, each at about 12,000 G and about 4°C for about 30 minutes, conditions to maintain the exosomes in intact form, to remove additional fat and debris. A substantially clear whey fraction was obtained. The substantially clear whey fraction was microfiltered using a 0.22 µm filter of hydrophilic polyethersulfone, and the microfiltered whey was then centrifuged at 100,000 G at 4°C for about 60 minutes to obtain an exosome-containing pellet. The exosome-containing pellet was suspended in sterile PBS (137 mM NaCl, 2.7 mM KCI, 8 mM Na2HP04, and 2 mM KH2P04; pH 7.4) or sterile molecular biology grade water in a centrifugation tube, which was then incubated for 12-36 hours in an orbital shaker at 4°C and 150 rpm. Notably, when the orbital shaking step was not utilized, aggregates appeared that required extensive pipetting to break up, leading to exosome bilayer membrane damage.

The exosomes were first frozen at -80°C for at least 2 hours and the frozen exosomes were then freeze dried under conditions which maintain the exosomes in intact form. Specifically, the step of freeze-drying comprised exposing the frozen exosomes to a temperature of -80°C and a vacuum of less than 0.3 mbar for a sufficient time period, about 24 hours, to ensure a low moisture content.

Importantly, thawing the exosomes prior to the vacuum stage of the freeze-drying led to milk exosome membrane damage. Therefore, embodiments of methods described herein comprise freezing the milk exosomes and keeping the milk exosomes frozen until freeze-drying is complete and powdered milk exosomes result. The isolation and drying conditions described herein maintain the integrity of the exosome bilayer membrane and the contents of the exosomes.

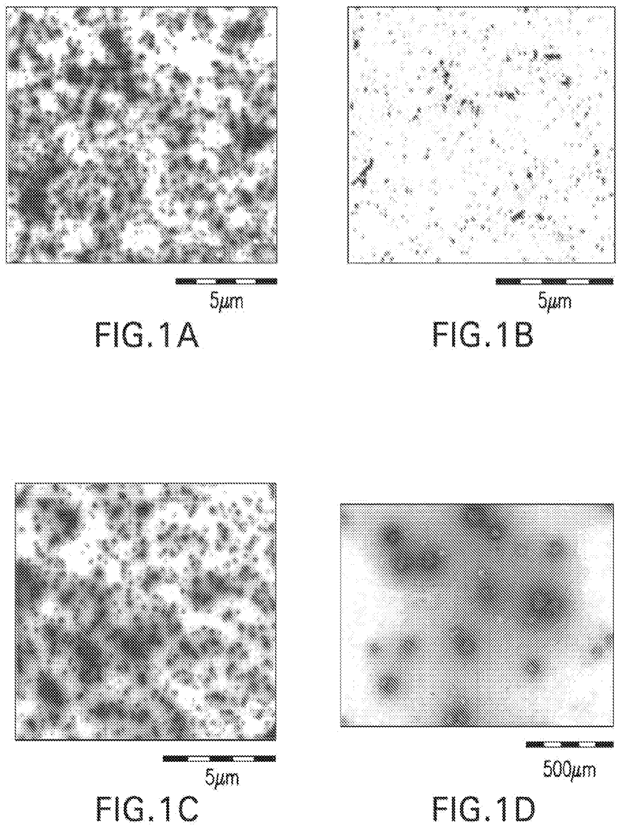

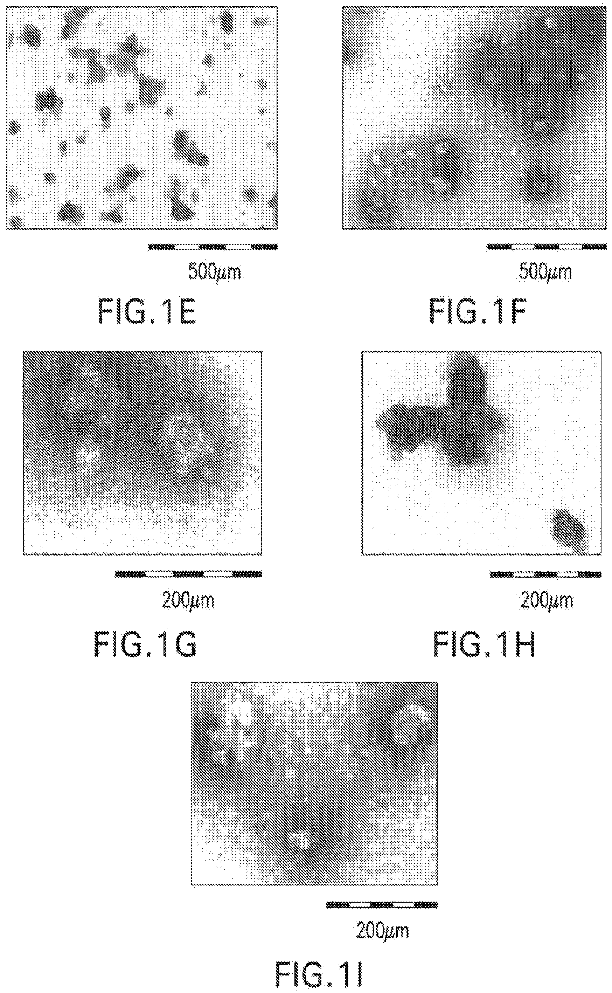

Milk exosome samples were visualized under a TEM at a magnification of 800x, as shown in FIGS. 1A-1C, at 10,000x as shown in FIGS. 1D-1F, and at 25,000x as shown in FIGS. 1G-1I. For comparison purposes, TEM analysis included fresh milk exosomes (**FIGS. 1A, 1D** **and** **1G**), milk exosomes frozen at -80°C for 2 hours and thawed on ice (**FIGS. 1B****,** **1E and 1H**), and powdered, freeze-dried milk exosomes according to a specific embodiment of the invention (**FIGS. 1C****,** **1F and 1I**). For the TEM images, each sample was stained with uranyl acetate, which is an electrodense compound. When the exosome membrane is damaged, uranyl acetate penetrates and stains the inner compartment. As readily shown by a comparison of FIG. 1G and FIG. 1I, freeze-drying did not alter the exosome structure (morphology, size or membrane integrity) of the powdered exosome (FIG. 1I) as compared with the fresh exosomes (FIG. 1G). In these figures, it is evident that the membrane integrity has not been compromised as uranyl acetate covers the exosome and the vesicle contour is highlighted by a dark shadow, while the inner portion of the vesicle remains pale. However, freezing and thawing the exosomes resulted in massive exosome membrane damage (**FIG. 1H**), where uranyl acetate penetrated and stained the inner compartment of the exosomes through the damaged membrane. Therefore, powdered exosomes provide a product in which the milk exosome membrane structure is preserved.

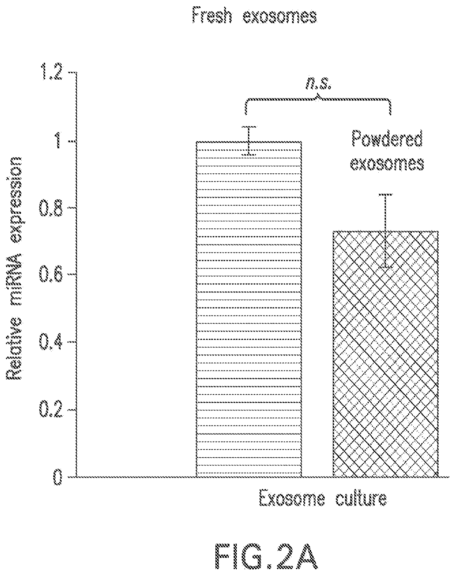

Freeze-dried exosomes also retained miRNA. To analyze miRNA content, fresh milk exosomes and powdered exosomes of the invention were resuspended in sterile PBS, and 60 microliters of each sample were used to isolate and quantify the miRNA content. miRNA isolation was performed using a commercially available kit (Invitrogen^{™} mirVana^{™} miRNA Isolation Kit, reference number AM 1560) following the manufacturer's protocols and technical recommendations. miRNA quantification was performed by microfluidic electrophoresis in an Agilent 2100 Bioanalyzer System using a Small RNA chip. FIG. 2A shows the miRNA-200c levels in the fresh milk exosomes and the powdered exosomes. This RNA (miRNA-200c) is abundant in bovine milk, and is found mainly within the whey fraction containing the exosomes. There was no statistical difference (p > 0.05, n.s. of FIG. 2A), between the level of miRNA-200c in fresh exosomes versus the level in the powdered exosomes. The miRNA-200c level from exosomes subjected to freezing and thawing is not shown due to the extensive damage previously recognized and shown in FIG. 1H.

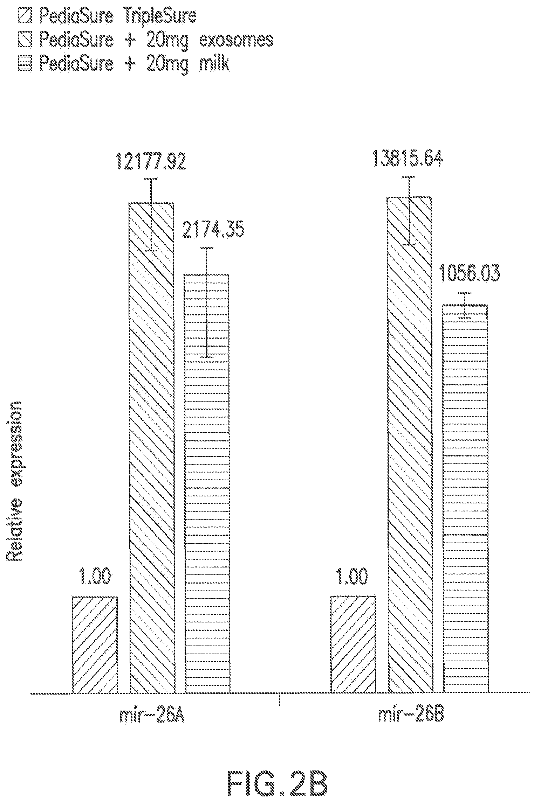

In addition, freeze dried raw milk and powdered exosomes according to the invention, respectively, were added to liquid nutritional products and certain miRNA levels were measured. Specifically, 20 mg of either freeze dried raw milk or the powdered exosomes according to the invention were added to 1 mL of commercially available PediaSure^{®} TripleSure^{™} nutritional compositions. The levels of miR-26a and miR-26b were analyzed. These miRNAs are of particular relevance regarding glucose metabolism. The results are shown in FIG. 2B, and show increased levels of the indicated miRNAs in the nutritional composition to which the powdered exosomes were added. These results demonstrate that the powdered exosomes preserved the structure and the content of the milk exosomes, the powdered exosomes are suitable for increasing miRNA levels in liquid nutritional products, and addition of the powdered exosomes provide miRNAs at a higher level than the freeze dried raw milk addition.

In a further experiment, the thermal stability of powdered exosomes according to the invention was evaluated. Both powdered exosomes prepared as described above and fresh milk exosomes were stored for 4 weeks under vacuum-packaging at either 25°C, 37°C or 55°C. Samples were analyzed at 1, 2, 3 and 4 weeks and compared with their respective thermally untreated controls. The levels of the exosome-borne specific miRNA (miR-200c) were determined in the exosomes. miRNAs are particularly sensitive to high temperatures and degradative enzymes. In order to prevent microbial growth during the experiment time course, fresh milk exosomes were treated with sodium azide (final concentration = 0.15% w/v) after confirming that sodium azide addition does not interfere with downstream sample analysis, nor does it disrupt the milk exosome structure. Total small RNA was extracted with the mirVana^{™} miRNA Isolation kit following the manufacturer's instructions. An external spike-in control oligonucleotide (cel-miR-39, AGCUGAUUUCGUCUUGGUAAUA) was added during sample preparation in order to monitor the efficiency of RNA extraction. In addition to that, cel-miR-39 was used to normalize potential sample-to-sample and technical variations.

The results are set forth in Table 1 and demonstrate that, regardless of the temperature tested, the powdered exosomes protected the miRNA content. Conversely, miR-200c levels statistically decreased when fresh milk exosomes were stored at 37°C and 55°C. That decrease was particularly relevant at 55°C, where only small amounts of miR-200c were detected after 1 week and no miR-200c was detected from 2 weeks forward. That decay of the miR-200c signal can be attributed to exosome breakage due to thermal damage.

**Table 1: Thermal Stability**

| | 25°C | | 37°C | | 55°C | |
|---|---|---|---|---|---|---|
| | Fresh Milk Exosomes | Powdered Milk Exosomes | Fresh Milk Exosomes | Powdered Milk Exosomes | Fresh Milk Exosomes | Powdered Milk Exosomes |
| Weeks | *miR-200c relative expression* | | *miR-200c relative expression* | | *miR-200c relative expression* | |
| 0 | 1 | 1 | 1 | 1 | 1 | 1 |
| 1 | 1.13 | 1.12 | 1.09 | 0.89 | 0.18* | 1.27 |
| 2 | 1.12 | 1.07 | 0.96 | 0.99 | 0* | 0.92 |
| 3 | 0.91 | 0.71 | 0.62* | 0.84 | 0* | 0.96 |
| 4 | 1.01 | 1.15 | 0.57* | 0.69 | 0* | 1.19 |

| | | | | | | |
|---|---|---|---|---|---|---|
| * Indicates statistical significance vs Week 0 (p<0.05) | | | | | | |

The results in Table 1 show that the powdered exosomes according to the invention exhibit good stability under high thermal conditions. This stability shows that the powdered exosomes are advantageous for an easy oral administration route, either directly or by addition to a nutritional composition.

### Example 2: Therapeutic Results

This example demonstrates that powdered exosomes, prepared as described above and shown to be intact, were taken up by muscle cells. This was shown by labeling the exosomes with a red dye, incubating the exosomes with L6.C11 muscle cells, and looking at the cells via microscopy. A control comprising L6.C11 muscle cells incubated with BSA labelled with the same red dye confirmed that the uptake was not promoted by the dye itself.

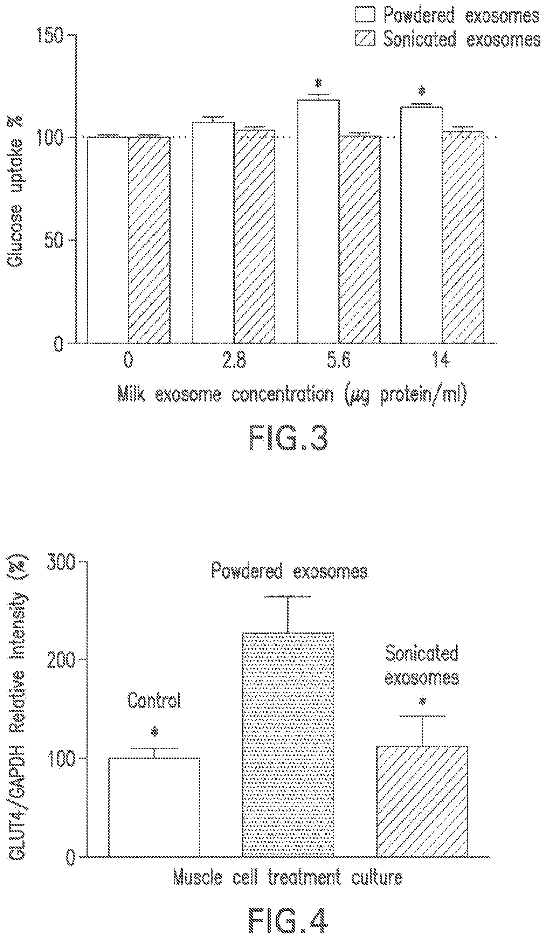

The powdered exosomes lead to an increased uptake of glucose by muscle cells. Powdered exosomes were added to L6.C11 muscle cell cultures until milk exosome concentrations of 0, 2.8, 5.6 and 14 µg/ml, respectively, were reached, and the cultures were incubated for 24 hours. Uptake of radiolabeled glucose was then assessed. The results of these incubations are shown via the white bars of FIG. 3. The concentration of 0 µg/ml (no exosomes) served as a control where glucose uptake was set at 100%. The concentrations of 5.6 and of 14 µg/ml powdered exosomes, respectively, led to a statistically significant increase in glucose uptake by the muscle cells versus the control (p <0.05). A comparative experiment was conducted in which the powdered exosomes were sonicated prior to the incubation. The black bars of FIG. 3 show the results of this comparative experiment. Sonication allowed for physical membrane disruption. In all cases, the sonication led to disruption of the exosome membrane and negated the positive effects of the powdered exosomes.

The increased uptake of glucose is in part due to an increase in GLUT-4 transporter expression. In another experiment, the powdered exosomes were added to a L6.C11 muscle cell culture at a concentration of 14 µg/ml, and the cell culture was incubated for 24 hours. A control culture without powdered exosomes was included, as was a culture of powdered exosomes at a concentration of 14 µg/ml where the powdered exosomes were also sonicated prior to the addition to the culture. Western blotting was used to assess protein expression for the GLUT-4 transporter. Glyceraldehyde-3-phosphate dehydrogenase (GADPH), a key enzyme involved in glycolysis, was used as a loading control, and the relative intensity of expression (level of GLUT-4 / GADPH) in the control culture (0 µg/ml) was set at 100%. The results are shown in **FIG. 4****.** There was a statistically significant difference between the control culture and powdered exosome culture, as GLUT-4 transporter expression was increased significantly in the powdered exosome culture. This effect was negated by sonication, as there was also a significant difference between the sonicated exosome culture and the powdered exosome culture.

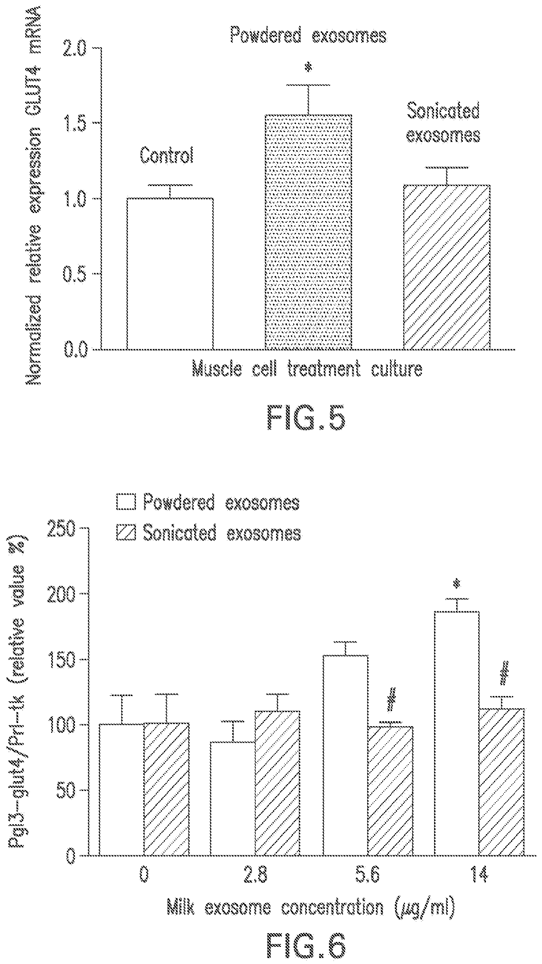

The same experimental design was used to assess whether the powdered exosomes were leading to an increase in GLUT-4 RNA as assessed via PCR and to determine whether the powdered milk exosomes were affecting translation as well as protein levels, and the results are shown in **FIG. 5****.** Normalized, relative expression of GLUT-4 mRNA in the powdered exosome culture was much higher than the expression in the control culture, and there was a statistically significant difference between the powdered exosome culture and the control culture. Sonicating the exosomes appeared to partially negate this positive effect, though there was no statistically significant difference between the powdered exosome culture and the sonicated exosome culture.

The powdered milk exosomes also increase GLUT-4 promoter activity, leading to an increase in overall GLUT-4 expression on the muscle cells, providing a mechanism for increased overall glucose uptake by the muscle cells. This was shown with the following experiment. Muscle cells were transfected with pGL3-GLUT-4 plasmid, which allowed for the expression of luciferase (fluorescent protein) under the control of a native GLUT-4 promoter. Then cells were incubated for 24 h with 0, 2.8, 5.6 and 14 µg/ml of powdered exosomes or sonicated exosomes. After the incubation period, fluorescence was measured, which was correlated to GLUT-4 promoter activity. The results are shown in **FIG. 6**. The relative value (Pgl3-glut4 / control reporter plasmid Prl-tk) / GADPH) in the control culture (0 µg/ml) was set at 100%. At 14 µg/ml, powdered exosomes significantly increased GLUT-4 promoter activity, and there was a statistically significant difference between the powdered exosome culture (14 µg/ml) and the control culture. There was also a statistically significant difference between the powdered exosome culture (14 µg/ml) and the corresponding sonicated exosome culture.

In summary, the powdered exosomes of the invention, prepared by gentle processing and drying so as not to not disrupt the milk exosome bilayer membrane structure, are superior to isolated exosomes which have been frozen and thawed, which leads to exosome membrane damage. Sonication also negates the positive effects of the powdered exosomes on muscle cells. Preservation of the exosome structure is important in achieving a biological effect. The powdered milk exosomes are taken up by muscle cells and increase glucose uptake in muscle cell by enhancing the transcription of the GLUT-4 gene. Further, increasing glucose uptake in muscle cells provides a method for enhancing control of glucose levels and muscle function in insulin resistant subjects by improving insulin sensitivity. Therefore, in specific embodiments, the powdered milk exosomes and/or a nutritional composition containing the powdered milk exosomes are administered to increase GLUT-4 transporter expression in a muscle of a subject. Further, in specific embodiments, the powdered exosomes and/or a nutritional composition to which the powdered exosomes are added is administered to a subject to lower a risk of developing insulin resistance, prediabetes, or diabetes in the subject, or to treat insulin resistance, prediabetes, or diabetes in the subject.

The specific embodiments and examples described herein are exemplary only and are not limiting to the invention defined by the claims.
The invention will now be described further in the following numbered embodiments:
1. Bovine milk-isolated powdered exosomes comprising intact exosomes.
2. The bovine milk-isolated powdered exosomes of embodiment 1, wherein the intact exosomes comprise miRNA.
3. The bovine milk-isolated powdered exosomes of embodiment 2, wherein, after storage at 37°C for 3 weeks, the bovine milk-isolated powdered exosomes comprise a greater amount of miRNA than fresh milk exosomes stored at 37°C for 3 weeks.
4. The bovine milk-isolated powdered exosomes of any one of embodiments 1-3, wherein the bovine milk-isolated powdered exosomes comprise freeze dried intact exosomes.
5. The bovine milk-isolated powdered exosomes of any one of embodiments 1-4, wherein greater than 90% of the exosomes are from about 10 nanometers to about 250 nanometers in diameter.
6. A nutritional composition comprising protein, carbohydrate, and/or fat, and bovine milk-isolated exosomes, wherein the exosomes are provided by addition of the powdered exosomes of any one of embodiments 1-5.
7. The nutritional composition of embodiment 6, comprising from about 0.001 to about 10 wt % of the bovine milk-isolated exosomes, based on the weight of the nutritional composition.
8. The nutritional composition of embodiment 6 or 7, wherein the protein comprises milk protein and/or soy protein.
9. The nutritional composition of any one embodiments 6-8, wherein the carbohydrate comprises fiber.
10. The nutritional composition of any one of embodiments 6-9, wherein the fat comprises at least one omega-3 fatty acid.
11. The nutritional composition of embodiment 10, wherein the at least one omega-3 fatty acid of the composition is selected from the group consisting of eicosapentaenoic acid, docosahexaenoic acid, arachidonic acid, and alpha-linolenic acid.
12. The nutritional composition of any one of embodiments 6-11, wherein the nutritional composition is in the form of a powder.
13. The nutritional composition of any one of embodiments 6-11, wherein the nutritional composition is in the form of a liquid.
14. A method of preparing a powdered nutritional composition, comprising dry blending a powder composition comprising protein, carbohydrate, and/or fat with the powdered exosomes of any one of embodiments 1-5.
15. A method of preparing powdered exosomes isolated from bovine milk, comprising:
   centrifuging bovine milk to form a lipid fraction top layer, a whey fraction middle layer, and a first pellet of cells and debris;
   separating the whey fraction and centrifuging the separated whey fraction to remove additional fat, casein aggregates and debris and form a substantially clear whey fraction;
   microfiltering the substantially clear whey fraction to remove residual debris;
   centrifuging the microfiltered whey fraction to obtain a pellet containing exosomes;
   incubating the exosome-containing pellet in aqueous medium to dissolve the pellet without disrupting exosome membranes to provide an exosome suspension; and
   drying the suspension to obtain the powdered exosomes.
16. The method of embodiment 15, wherein the incubation step is conducted for about 12 to about 36 hours and at not more than 500 rpm centrifugation.
17. The method of embodiment 15 or embodiment 16, wherein the drying step comprises freeze drying.
18. The method of embodiment 17, wherein the freeze-drying comprises exposing the exosome suspension to a temperature of -80°C and a vacuum of less than 0.3 mbar for at least 24 hours.
19. The method of any one of embodiments 15-18, wherein the method is conducted with bovine milk within about 5 days of obtaining the milk from a bovine, or wherein the bovine milk is thawed from bovine milk that was frozen within about 5 days of obtaining the milk from a bovine.
20. A method of lowering a risk of developing insulin resistance, prediabetes, or diabetes in a subject, or treating insulin resistance, prediabetes, or diabetes in a subject, comprising:
   administering to a subject at risk of developing, or having, insulin resistance, prediabetes, or diabetes the powdered exosomes of any one of embodiments 1-5 or the nutritional composition of any one of embodiments 6-13, wherein the administered amount of exosomes is effective to lower the risk of development of, or treat, respectively, insulin resistance, prediabetes, or diabetes.
21. The method of embodiment 20, wherein the subject is obese or has sarcopenia.

## Claims

1. Bovine milk-isolated powdered exosomes for use in lowering a risk of developing insulin resistance, prediabetes, or diabetes in a subject, or treating insulin resistance, prediabetes, or diabetes in a subject, the bovine milk-isolated powdered exosomes comprising intact exosomes.

2. The bovine milk-isolated powdered exosomes for use of claim 1, wherein the subject is obese or has sarcopenia.

3. A nutritional composition for use in lowering a risk of developing insulin resistance, prediabetes, or diabetes in a subject, or treating insulin resistance, prediabetes, or diabetes in a subject, the nutritional composition comprising protein, carbohydrate, and/or fat, and bovine milk-isolated powdered exosomes comprising intact exosomes.

4. The nutritional composition for use of claim 3, wherein the nutritional composition comprises protein, carbohydrate, and fat.

5. The nutritional composition for use of claim 3 or claim 4, wherein the nutritional composition comprises protein in an amount from about 1 wt% to about 30 wt% of the nutritional composition.

6. The nutritional composition for use of any one of claims 3-5, wherein the nutritional composition comprises carbohydrate in an amount from about 5 wt% to about 70 wt% of the nutritional composition.

7. The nutritional composition for use of any one of claims 3-6, wherein the nutritional composition comprises fat in an amount from about 1 wt% to about 30 wt% of the nutritional composition.

8. The nutritional composition for use of any one of claims 3-7, wherein the nutritional composition comprises milk protein concentrate and/or soy protein isolate as proteins; canola oil, sunflower oil, medium chain triglycerides, and/or DHA as fats; and hydrolyzed corn starch, sucrose, and/or maltodextrin as carbohydrates.

9. The nutritional composition for use of any one of claims 3-7, wherein the nutritional composition comprises milk protein concentrate as protein; corn maltodextrin, fructose, and/or glycerine as carbohydrates; and high oleic safflower oil as fat.

10. The nutritional composition for use of any one of claims 3-9, wherein the nutritional composition has a caloric density of about 0.5 to about 3 kcal/mL.

11. The nutritional composition for use of any one of claims 3-10, wherein the nutritional composition comprises β-hydroxy-β-methylbutyrate (HMB), preferably wherein the nutritional composition comprises HMB in an amount from about 0.01 wt% to about 10 wt% of the nutritional composition.

12. The nutritional composition for use of any one of claims 3-11, wherein the composition is in powdered or liquid form.

13. The bovine milk-isolated powdered exosomes for use of claim 1 or claim 2, or the nutritional composition for use of any one of claims 3-12, wherein the bovine milk-isolated powdered exosomes or the nutritional composition is for administration to the subject from 1 to 4 times per day.

14. The bovine milk-isolated powdered exosomes for use of any one of claims 1, 2 or 13, or the nutritional composition for use of any one of claims 3-13, wherein administration to the subject increases an uptake of glucose by muscle cells of the subject.

15. The bovine milk-isolated powdered exosomes for use of any one of claims 1, 2, 13, or 14, or the nutritional composition for use of any one of claims 3-14, wherein administration to the subject increases GLUT-4 transporter expression in a muscle of the subject.

16. The bovine milk-isolated powdered exosomes for use of any one of claims 1, 2 or 13-15, or the nutritional composition for use of any one of claims 3-15, wherein the powdered exosomes or the nutritional composition is for use in enhancing muscle function in the subject, wherein the subject has insulin resistance.
